# EUROPEAN PATENT APPLICATION

(11) **EP 2 777 495 A1**
(43) Date of publication of application: **17.09.2014**
(21) Application number: 14157299.0
(22) Date of filing: 28.02.2014
(51) Int. Cl.: A61B 5/0408, A61N 1/04

(54) **Reduce motion artifact electrode**

(30) Priority: 15.03.2013 US 201361792760 P
(71) Applicant: Covidien LP, Mansfield, MA 02048 (US)
(72) Inventor: Meyer, Peter, Shrewsbury, MA Massachusetts 01545 (US); Selvitelli, David, Suffield, CT Connecticut 06078 (US)
(74) Representative: Olsson, Carl H.S.

(57) **Abstract**

A medical electrode which mechanically isolates a conductive interface between the electrode and a patient's skin from forces applied to the lead wires or a lead wire connector is described.

## Description

### CROSS REFERENCE TO RELATED APPLICATIONS

Not Applicable.

### STATEMENT REGARDING FEDERALLY SPONSORED RESEARCH

Not Applicable.

### FIELD OF THE INVENTION

The concepts, circuits and techniques described herein relate generally to medical electrodes and, more particularly, to medical electrodes for biosignal acquisition or electrical stimulation.

### BACKGROUND OF THE INVENTION

As is known in the art, conventional medical electrodes generally include a conductive electrode member and an electrical conductor that provides an electrical interface between the electrode and various types of medical equipment, such as monitoring equipment, signal acquisition equipment and stimulating equipment. Illustrative monitoring equipment includes electrocardiograph (ECG) monitors and illustrative stimulating equipment includes transcutaneous stimulation equipment and defibrillators.

As is also known, disposable medical electrode pads often include a pad member surrounding an electrode. An adhesive material is disposed on a bottom surface of the pad except for a central portion which contains a conductive hydrogel for making good electrical contact with a patient's skin when the pad is pressed in place. A cable terminating in an electrode connector is connected to a conductor on the pad so as to form an appropriate electrical connection.

Once the appropriate signals have been recorded, the electrode connectors are removed from the conductors on the electrode pad and the pad is then removed from the patient and thrown away.

As is also known, motion artifacts (e.g. spurious signals) in ambulatory recordings reduce the reliability of tests (e.g. Holter recordings and stress tests). This is particularly true when such tests are computer assisted. Motion artifacts can also cause false alarms during patient monitoring, which can reduce clinician confidence in monitoring equipment alarms and, consequently, slow response time.

One source of motion artifacts (e.g. in ECG monitoring) is relative motion between the electrode and the electrode connector. For example, motion artifacts can be generated as a result of lateral forces applied to an electrode or a lead wire coupled to the electrode and also due to rotation of a lead wire with respect to an electrode connection point such as a stud.

To reduce motion artifacts, one class of medical electrodes (e.g. Medico Lead-Lok electrodes), requires a clinician to thread each lead wire through a slit in the corresponding electrode. While such an approach helps prevent rotation of the lead wire with respect to the electrode stud, this approach fails to provide an amount of mechanical isolation from lateral forces applied to the lead wire or electrode sufficient to eliminate or even reduce motion artifacts below desired levels.

Furthermore, while the Medico Lead-Lok electrode is a relatively simple, inexpensive electrode, it requires a clinician or other person to thread each lead wire through a slit in the corresponding electrode. If the electrode must be replaced, it can be difficult to detach the electrode from the lead wire due to an adhesive coupling between the lead wire and the adhesive foam of the electrode.

### SUMMARY OF THE INVENTION

In accordance with the concepts, systems, circuits and techniques described herein, a medical electrode includes a substrate having a connector portion with a connector connected thereto, a patient contact portion having a conductive pad coupled thereto and an isolating portion which provides a flexible mechanical connection between the first and second substrate portions and which also provides a flexible electrical connection between the connector and the patent contact portion of the substrate. The connector is configured to couple to a lead wire which may be coupled to various types of medical equipment. The conductive pad on the patient contact portion of the substrate is adapted to provide a conductive interface between the connector and a patient's skin.

With this particular arrangement, a medical electrode which mechanically isolates a conductive interface to connector the patient's skin from forces applied to an electrode connector or lead wires is provided. The substrate is disposed over a dielectric member (e.g. a foam pad) having an adhesive disposed on at least portions of a surface thereof such that the dielectric member can be non-conductively attached to a patient's skin.

In some embodiments, the connector may be provided as a stud, a snap, a spring loaded clip (e.g. a so-called "alligator clip") a pre-wired connection or any other structure suitable to provide an electrical connection to a lead wire. The conductive pad on the patient contact portion of the substrate may be provided from a conductive ink printed or otherwise disposed over the substrate. A conductive adhesive hydrogel pad may be disposed over the conductive pad to secure the patient contact portion of the substrate to the patient's skin and provide an electrical connection between the patient's skin and the electrical conductor.

The connector (e.g. stud) preferably has a connection to a patient's skin (e.g. via the foam adhesive pad) which is more rigid than the connection between the connector and the conductive pad on the patient contact portion of the substrate.

The isolating portion of the substrate provides a flexible conductive interface between the connector and the conductive pad. The isolating portion of the substrate is preferably provided having an elasticity characteristic greater than the elasticity characteristic of the connector portion such that connector portion of the substrate is mechanically isolated from the patient contact portion of the substrate. The isolating portion of the substrate includes a conductive signal path configured to provide a flexible electrical connection between the electrical contact (e.g. a stud) and the conductive pad. In one embodiment, the conductive signal path which provides the flexible electrical connection is provided from a conductive ink printed or otherwise disposed on the substrate. The conductive signal path is provided having a relief region which helps mechanically isolate the connector from the conductive pad. In one exemplary embodiment, the relief region is provided by the conductive signal path having a shape which allows the conductive signal path to expand and contract with the isolating portion of the substrate while maintaining an electrical connection between the electrical contact (e.g. a stud) and the conductive pad. In one embodiment, the conductive signal path may be provided having a meanderline shape, a serpentine shape or any other shape which allows movement (e.g. of lead wires or a stud) while substantially mechanically isolating the electrical pad from such movement. It should, of course, be appreciated that the conductive signal path may also be provided from materials other than conductive ink so long as an electrical connection between the connector and the conductive pad is maintained during movement of all or any portion of the medical electrode as well as during movement of lead wires or other circuits, systems and/or devices coupled to the medical electrode (e.g. via one or more lead wires).

In some embodiments, the connector substrate portion may be provided having a ring shape (e.g. a ring-shaped pad or foam) which surrounds or partially surrounds the patient contact substrate portion. The ring-shape may be provided as any regular or irregular geometric shape including but not limited to an oval shape, a circular shape, a rectangular shape, a square shape, a triangular shape, or any other mutli-sided shape (e.g octagonal shape) or any other regular or irregular shape. In some embodiments, the connector substrate portion may lie proximate or adjacent the patient contact substrate portion (rather than fully or partially surrounding the second substrate portion). Likewise, the patient contact and the isolating portions of the substrate may also be provided having any regular or irregular geometric shape including but not limited to an oval shape, a circular shape, a rectangular shape, a square shape, a triangular shape, or any other mutli-sided shape (e.g. an octagonal shape) or any other regular or irregular shape.

In one embodiment, the connector (e.g. stud) preferably has a connection to the foam pad (and thus to a patient's skin) which is more rigid than the connection between the stud and the conductive pad (e.g. the conductive ink which makes contact with the patient's skin through a conductive adhesive hydrogel). In one embodiment, the stud is securely coupled to a foam ring through a flexible substrate and the stud is flexibly coupled to the electrical contact through a conductor having a relief region and disposed on the isolating portion of the substrate.

In accordance with a further aspect of the concepts, systems, circuits and techniques described herein, a medical electrode includes a substrate having conductive ink printed or otherwise disposed thereon. Conductive ink makes electrical contact with a patient's skin through a conductive adhesive hydrogel pad. Portions of the conductive ink that are not intended to contact hydrogel are covered by a dielectric material. The substrate is disposed over and non-conductively connected to the patient via an adhesive foam. The device may be connected to a lead wire (e.g. an ECG lead wire) through a connector such as a stud or similar means known in the art. Prior to activation, the substrate is suspended above the patient skin by the foam. In one embodiment, the foam is provided in the shape of a ring. An overhang region of foam ring prevents an otherwise cantilevered portion of substrate from falling onto the patient skin prior to activation. The medical electrode is activated by pressing down on a portion of substrate above the hydrogel pad. This disengages the cantilevered substrate from the foam overhang and allows the hydrogel pad to be affixed to the patient's skin. Slits (or other openings) provided in the substrate provide strain relief between the hydrogel engaging portion and the foam engaging portions of substrate. Once activated, the foam ring remains tightly coupled to the stud, while the hydrogel pad is flexibly coupled to the stud. Thus, forces applied to the stud are largely transmitted through the foam ring to the patient's skin surrounding the electrode site, with relatively little force being transmitted through the hydrogel to the patient's skin at the electrode contact site.

With this particular arrangement, an electrode intended for biosignal acquisition or electrical stimulation is provided. The device mechanically isolates the conductive interface between the electrode and the patient's skin from forces applied to the lead wires. Such forces may, for example, result from inertial motion of the lead wires induced by patient movement.

The medical electrode described herein provides mechanical isolation of the electrode/skin contact area, but in contrast to the prior art approaches, does not require a clinician to perform any additional steps to implement the mechanical isolation feature.

In accordance with the concepts, systems, circuits and techniques described herein, a method for fabricating a medical electrode includes a method for fabricating a medical electrode includes providing a dielectric member and disposing a substrate over the dielectric member, the substrate having first and second opposing surfaces and comprising a patient contact portion and a dielectric engaging portion wherein the dielectric member is disposed to suspended the substrate above the patient's skin when the substrate is in the first position. The method further includes providing a strain relief region in the substrate and securing a connector to the substrate. The strain relief region is located between the patient contact portion of the substrate and the connector such that the substrate is moveable between a first position in which the substrate is suspended above a patient's skin and a second position in which at least the patient contact portion of the substrate is in contact with the patient's skin.

The method further includes providing a conductive signal path on the substrate which electrically couples the connector to the patient contact portion of the substrate wherein the conductive signal path is provided having a shape that provides strain relief between the connector and the patient contact portion of the substrate (e.g. the characteristics of the conductive signal path are selected to allow the conductor to move in response to movement of the connector or the substrate. The strain relief regions of the substrate and the conductor operate so as to mechanically isolate the patient contact portion of the substrate from such movement while maintaining an electrical connection between the connector and the patient contact portion of the substrate.

It should be appreciated that the elements of the above-recited process are unordered meaning that they can be performed in any order which is logically possible.

In one embodiment, providing a strain relief region in the substrate comprises forming openings in the substrate. In one embodiment, the opening may be provided as slits or cuts in the substrate.

In one embodiment, providing a strain relief region in the conductor comprises providing a conductor having one of a meander line shape, a curved shape and a serpentine shape.

### BRIEF DESCRIPTION OF THE DRAWINGS

The foregoing features of the invention, as well as the invention itself may be more fully understood from the following detailed description of the drawings, in which:
Fig. 1 is a plan bottom view (patient contact side) of a medical electrode;
Fig. 2 is a side view of the medical electrode of Fig. 1 prior to activation and taken along lines 2-2 in Fig. 1;
Fig. 2A is a side view of the medical electrode of Fig. 1 post activation;
Fig. 2B is an enlarged side view of a portion of the medical electrode of Fig. 2 taken along lines 2B-2B in Fig. 2;
Fig. 3 is a plan bottom view (patient contact side) of an alternate embodiment of a medical electrode; and
Fig. 4 is a diagrammatic view of application of medical electrodes of the types described in Figs. 1-3 to a patient and connection of the electrodes to a medical device.

### DETAILED DESCRIPTION OF THE INVENTION

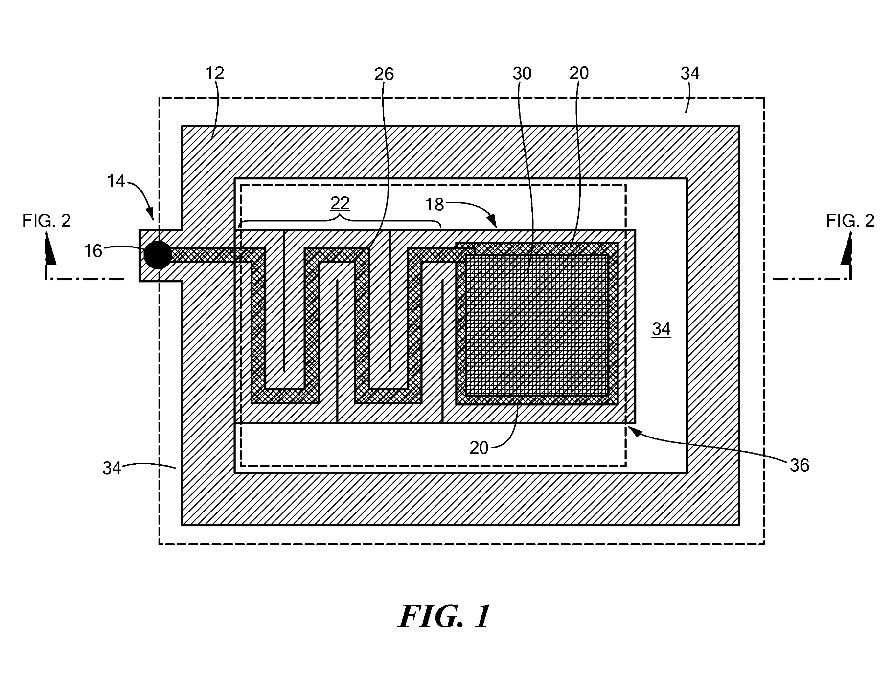

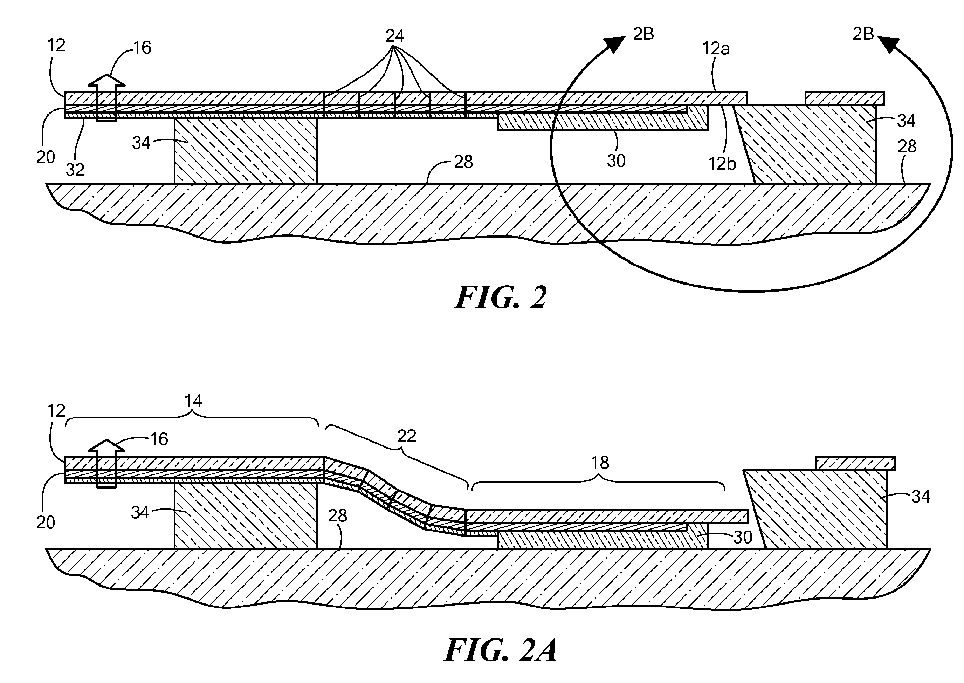

Referring now to Figs. 1 - 2B in which like elements are provided having like reference designations throughout the several views, a medical electrode 10 includes a substrate 12 having first and second opposing surfaces 12a, 12b (Fig. 2A). For reasons which will become apparent hereinbelow, the type and thickness of substrate 12 is selected such that the substrate is flexible. In one embodiment, substrate 12 may be provided from a Polyethylene Terephthalate (PET) resin (such as the type manufactured by Dupont Tejjin Films and marketed under the brand name Mylar®). Those of ordinary skill in the art will appreciate, of course, that substrate 12 may be provided from other materials such as other types of polyester films or plastic sheets.

Substrate 12 has a first or connector portion 14 with a connector 16 connected thereto and a second or patient contact portion 18 having a conductive pad 20 disposed thereon. Substrate 12 also includes an isolating portion 22 which provides a flexible mechanical connection between the first and second substrate portions 14, 18.

In this particular exemplary embodiment, isolating portion 22 of substrate 12 is made flexible via a plurality of here five (5), slits 24 which provide isolating portion of substrate 12 as a segmented substrate section. In some embodiments isolating potion 22 does not include slits. Rather, the substrate may be made flexible via selection of materials or through the introduction of joints in the substrate 12.

A conductive signal path 26 disposed on both the first substrate portion and the isolating substrate portion 22 electrically couples connector 16 to the conductive pad 20 disposed on the patient contact portion 18 of the substrate 12. Thus, isolating portion 22 provides both a flexible mechanical connection and a flexible electrical connection between the connector 16 on the first substrate portion and conductive pad 20 on the patient contact portion of the second 12.

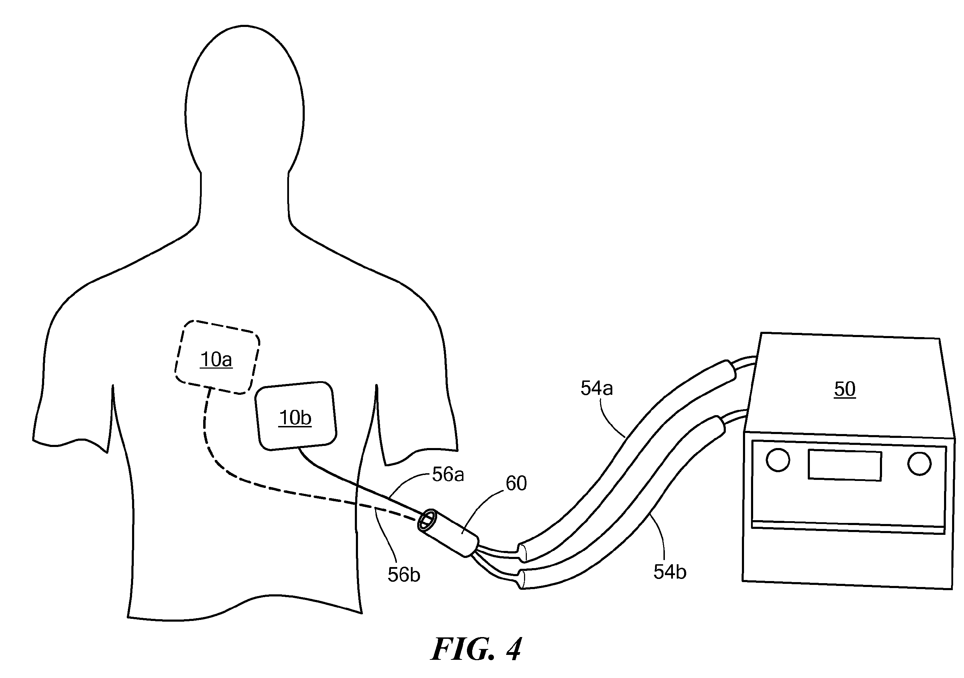

Connector 16 is configured to couple to lead wires (Fig. 4) and the conductive pad 20 on the patient contact portion of the substrate is adapted to provide a conductive interface to a patient's skin 28.

conductive signal path 26 and conductive pad 20 may be provided, for example, from a conductive material such as a conductive ink printed or otherwise disposed over the substrate 12 in a desired pattern. It should be noted that in the isolating portion 22 of substrate 12, conductive signal path 26 is provided having a meanderline pattern. Also, although conductive pad 20 is here shown having a substantially square shape, those of ordinary skill in the art will appreciate that conductive pad 20 may be provided having any regular or irregular geometric shape.

In the exemplary embodiment shown in Figs. 1-3, conductive pad 20 preferably makes electrical contact with a patient's skin 28 through a skin compatible conductive adhesive hydrogel pad 30 having good ability to retain moisture content and sufficient adhesive to adhesively secure the electrode to the patient's skin. Examples of suitable hydrogels include, but are not limited to, conductive hydrogels commercially available from the Kendall-LTP division of Tyco Healthcare Group LP, Mansfield, Mass., such as RG-63B conductive hydrogel. Hydrogel pad 30 is preferably provided having a shape corresponding to the shape of conductive pad 20.

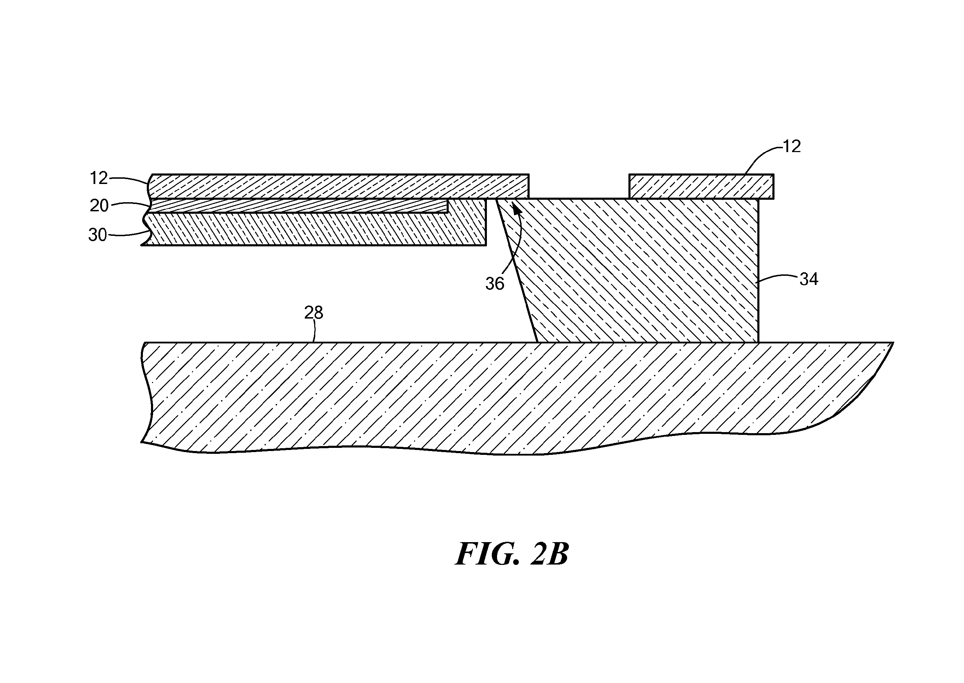

Portions of conductive material 26 that are not intended to contact hydrogel pad 30 are covered by a dielectric 32 (most clearly seen in Fig. 2).

Substrate 12 is disposed over an adhesive foam 34 and non-conductively coupled to a patient via the adhesive foam 34. The medical electrode is coupled for example, to an ECG machine via an ECG lead wire (Fig. 4) having a first end coupled to connector 16 or similar means known in the art.

As may be most clearly seen in Fig. 2, prior to activation, substrate 12 is suspended above the patient skin 28 by foam pad 34. In this exemplary embodiment, overhang 36 (Fig. 2B) of foam pad 34 prevents the otherwise cantilevered portion of substrate 12 from falling onto the patient skin 28 prior to activation. The medical electrode 10 is activated by pressing down on a portion of substrate 12 (preferably the portion of substrate 12 immediately above hydrogel pad 30). This disengages the cantilevered substrate 12 from foam overhang 36 and allows hydrogel pad 30 to be affixed to the patient's skin as illustrated in Fig. 2A.

The openings 24 (illustrated as slits 24 cut or otherwise provided in the substrate) provide strain relief between the hydrogel engaging portion and the foam engaging portions of substrate 12. Once activated, foam pad 34 remains tightly coupled to stud 16, while hydrogel pad 30 is flexibly coupled to stud 16 through isolating portion 22. Thus, forces applied to stud 16 are largely transmitted through foam pad 34 to the surface of the patient's skin surrounding the electrode site, with relatively little force being transmitted through the hydrogel pad 30 to the patient's skin at the electrode contact site.

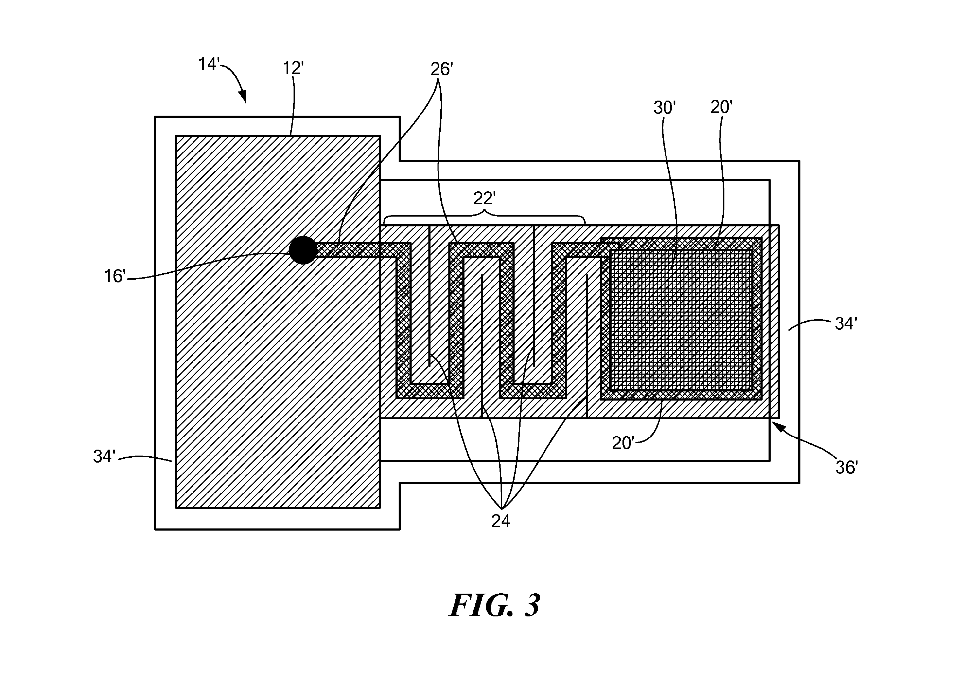

Referring now to Fig. 3, an alternate embodiment of a medical electrode includes a substrate 12' having a connector portion 14' adjacent an isolating portion or region 22'. Isolating region 22' couples the connector portion to a patient contact portion 18' of the substrate. A connector 16' disposed on the connector portion of the substrate is electrically and mechanically coupled to a conductive pad via a conductive signal path having a meander line shape. An adhesive hydrogel pad 30' is disposed over the conductive pad. Substrate 12', connector 16', conductive pad and hydrogel pad may be the same as or similar to those described above in conjunction with Figs. 1-2B.

The patient contact portion is here provided from a conductive pad having a hydrogel pad disposed thereover. The substrate is disposed over a dielectric member 34' having an overhang region (or a substrate engaging region) similar to overhang region 36 described above in conjunction with Figs. 1-2B. Dielectric member 34' may be provided as an adhesive backed dielectric foam. The relative sizes of the connector portion and patient contact portion are selected to promote mechanical isolation of patient contact portion from movement of the connector and/or movement of lead wires coupled to the connector. In particular, the connector portion has a larger surface area than the patient contact portion. Thus, connector portion is secured to a larger surface of the patient's skin than the patient contact portion. Consequently, once activated, connector portion is more securely connected to a patient's skin than the patient contact portion and the foam pad remains tightly .coupled to connector 16' while the hydrogel pad 30' is flexibly connected to the connector 16' through isolating portion 22'. Thus, forces applied to the connector are substantially transmitted through the foam pad to the patient's skin proximate the electrode site, with relatively little force being transmitted through the hydrogel pad to the patient's skin.

Referring now to Fig. 4, a medical device 50, as may take the form of an ECG monitor, a defibrillator, a transcutaneous stimulation device, an electrosurgical device, or a combination device as just a few examples, is connected through conductors 54a, 54b to a polarized connector (not visible in Fig. 4). Medical electrodes 10a', 10b' are positioned on the patient and may take the form of the medical electrodes described above in conjunction with Figs. 1-3.

Conductors 56a, 56b of respective electrodes 10a', 10b' terminate at a connector 60 that is adapted for coupling to the device connector as shown. Since the electrodes 10a', 10b' are X-ray transparent, they can be positioned on the patient at any customary position used for defibrillation for example without adversely affecting X-rays of the patient's chest in areas underlying the electrodes.

All references cited herein are hereby incorporated herein by reference in their entirety.

Having described preferred embodiments, which serve to illustrate various concepts, structures and techniques, which are the subject of this patent, it will now become apparent to those of ordinary skill in the art that other embodiments incorporating these concepts, structures and techniques may be used. Accordingly, it is submitted that that scope of the patent should not be limited to the described embodiments but rather should be limited only by the spirit and scope of the following claims.

## Claims

1. A medical electrode comprising:
a substrate having a connector portion, a patient contact portion and an isolating portion wherein the isolating portion provides a flexible mechanical connection between the connector and patient contact portions of said substrate;
a connector coupled to the connector portion of said substrate;
a conductive pad disposed on the patient contact portion of said substrate;
a conductive signal path disposed on said substrate to electrically couple said connector to said conductive pad and wherein said conductive signal path is provided having a shape selected to provide a flexible electrical connection between said connector and said conductive pad.

2. The medical electrode of claim 1 wherein the isolating portion mechanically isolates the conductive pad from forces applied to the connector.

3. The medical electrode of claim 1 wherein the isolating portion of said substrate is provided from a material having an elasticity characteristic which allows said substrate to stretch.

4. The medical electrode of claim 1 wherein conductive signal path disposed on the isolating portion of said substrate is configured to provide a flexible electrical connection between said electrical contact on the connector portion of said substrate and the conductive pad on the patient contact portion of said substrate.

5. The medical electrode of claim 4 wherein the conductive signal path disposed on the isolating portion of said substrate is provided having one of a meander line shape, a curved shape or a serpentine shape.

6. The medical electrode of claim 4 wherein said conductive signal path which provides the flexible electrical connection on the isolating portion of said substrate is provided from a conductive ink disposed on a surface of said substrate.

7. The medical electrode of claim 4 wherein said conductive signal path is provided having a shape which allows the conductor to expand and contract with the isolating portion of said substrate while maintaining an electrical connection between said connector and said conductive pad.

8. The medical electrode of claim 1 wherein said connector on the connector portion of said substrate is configured to couple to lead wires and said conductive pad is adapted to provide a conductive interface to a patient's skin.

9. The medical electrode of claim 8 wherein said connector is provided as one of a stud, a snap, a spring loaded clip or a pre-wired connection.

10. The medical electrode of claim 1 wherein said conductive pad is provided from a conductive ink disposed over a surface of said substrate and the medical electrode further comprises a conductive adhesive hydrogel disposed over said conductive pad to secure said conductive pad to the patient's skin and provide an electrical connection between the patient's skin and the conductive pad.

11. The medical electrode of claim 1 further comprising a dielectric member having a first surface over which said substrate is disposed and having a second surface having an adhesive disposed on at least portions thereof such that said dielectric member can be attached to a patient's skin.

12. The medical electrode of claim 11 wherein said connector is mechanically coupled to a patient's skin via said foam adhesive pad with a connection which is more rigid than a mechanical connection between said connector and said conductive pad provided by isolating portion of said substrate.

13. The medical electrode of claim 1 wherein the connector portion of said substrate is provided having a ring shape which surrounds or partially surrounds at least a portion of the patient contact portion of said substrate.

14. The medical electrode of claim 1 wherein the connector portion of said substrate is disposed adjacent the patient contact portion of said substrate.

15. A medical electrode comprising:
a substrate having first and second opposing surfaces and comprising a patient contact portion, said substrate moveable between a first position in which said substrate is suspended above a patient's skin and a second position in which at least the patient contact portion of said substrate is in contact with the patient's skin;
a dielectric member disposed to suspend said substrate above the patient's skin when said substrate is in the first position;
a connector, disposed on said substrate, said connector adapted to couple to a lead wire; and
a conductive signal path disposed on said substrate, said conductive signal path having a first end electrically coupled to said connector and a second end electrically coupled to the patient contact portion of said substrate, said conductive signal path having a shape selected to provide strain relief between said connector and the patient contact portion of said substrate so as to mechanically isolate the patient contact portion of said substrate from movement while of said connector and while maintaining an electrical connection between said electrical contact and the patient contact portion of said substrate.

16. The medical electrode of claim 15 wherein the patient contact portion of said substrate comprises a conductive ink disposed on a first one of the first and second opposing surfaces said substrate and a conductive adhesive hydrogel pad disposed over said conductive ink such that said conductive ink makes electrical contact with the patient's skin through said conductive adhesive hydrogel pad.

17. The medical electrode of claim 15 further comprising a dielectric sheet disposed over portions of said conductive ink that are not intended to contact said conductive adhesive hydrogel pad.

18. The medical electrode of claim 15 wherein said dielectric member is provided as an adhesive foam.

19. The medical electrode of claim 18 wherein said adhesive foam is provided as an adhesive foam having a ring-shape and said substrate is non-conductively connected to a patient's skin via said adhesive foam ring.

20. The medical electrode of claim 19 wherein said adhesive foam ring comprises an overhang portion which engages a portion of substrate proximate the patient contact portion of said substrate and prevents said substrate from falling onto the patient skin and wherein the medical electrode is activated by pressing down on a portion of said substrate to thereby disengage said substrate from said overhang portion and thereby allowing said conductive, adhesive hydrogel pad to be affixed to the patient's skin.

21. The medical electrode of claim 20 wherein the medical electrode is activated by pressing down on a portion of said substrate substantially above said conductive, adhesive hydrogel pad.

22. The medical electrode of claim 20 wherein said substrate has openings provided therein to provide strain relief between the patient contact portion of said substrate and said connector such that when said substrate is placed in its second position, said adhesive foam ring remains tightly coupled to said connector and said conductive, adhesive hydrogel pad is flexibly coupled to said connector such that forces applied to said connector are substantially transmitted through said adhesive foam ring to the patient's skin surrounding said conductive, adhesive hydrogel pad with relatively little force being transmitted through said conductive, adhesive hydrogel pad to the patient's skin at the site at which said conductive, adhesive hydrogel pad contacts the patient's skin.

23. A method for fabricating a medical electrode comprising:
providing a dielectric member;
disposing a substrate over the dielectric member, the substrate having first and second opposing' surfaces and comprising a patient contact portion and a dielectric engaging portion wherein the substrate is disposed over the dielectric member such that the dielectric member suspends the substrate above the patient's skin when the substrate is in a first position;
securing a connector to the substrate;
providing a strain relief region in the substrate, the strain relief region located between the patient contact portion of the substrate and the connector such that the substrate is moveable between the first position in which the substrate is suspended above a patient's skin and a second position in which at least the patient contact portion of the substrate is in contact with the patient's skin; and
providing a conductor on the substrate which electrically couples the connector to the patient contact portion of the substrate wherein the conductor is provided having a shape corresponding to a strain relief region that provides strain relief between the connector and the patient contact portion of the substrate wherein the strain relief regions of the substrate and the conductor operate so as to mechanically isolate the patient contact portion of the substrate from movement of the connector while maintaining an electrical connection between the connector and the patient contact portion of the substrate.

24. The method of claim 23 wherein providing a strain relief region in the substrate comprises forming openings in the substrate.

25. The method of claim 23 wherein a providing a strain relief region in the conductor comprises providing a conductor having one of a meander line shape, a curved shape and a serpentine shape.
